# EUROPEAN PATENT SPECIFICATION

(11) **EP 3 538 074 B1**
(45) Date of publication and mention of the grant of the patent: **26.07.2023**
(21) Application number: 17869130.9
(22) Date of filing: 10.11.2017
(51) Int. Cl.: A61K 9/127, A61M 25/00, A61P 35/00, A61K 31/337, A61K 33/24

(54) **METHODS OF TREATING UPPER TRACT UROTHELIAL CARCINOMAS**
VERFAHREN ZUR BEHANDLUNG VON UROTHELKARZINOMEN DES OBEREN TRAKTES
MÉTHODES DE TRAITEMENT DE CARCINOMES UROTHÉLIAUX DE LA VOIE EXCRÉTRICE SUPÉRIEURE

(30) Priority: 11.11.2016 US 201662421055 P
(43) Date of publication of application: 18.09.2019
(73) Proprietor: Western University Of Health Sciences, Pomona, CA 91766 (US); Tesorx Pharma, LLC, Menlo Park, CA 94025 (US)
(72) Inventor: BETAGERI, Guru, V., Chino Hills CA 91709 (US); VENKATESAN, Natarajan, Diamond Bar CA 91765 (US); OEFELEIN, Michael, G., Bakersfield, CA 93311 (US)
(74) Representative: Jacobi, Markus Alexander
(86) International application number: PCT/US2017/061047
(87) International publication number: WO 2018/089759

(56) References cited:
- WO-A1-2016/134066
- WO-A1-2016/151595
- WO-A1-2016/172343
- WO-A1-2017/120586
- JP-A- 2015 164 553
- US-A1- 2009 017 105
- US-A1- 2009 171 241
- US-A1- 2014 172 118
- US-B2- 9 445 995

## Description

### Field of the Invention

Methods for locally delivering a chemotherapeutic agent to an upper tract urothelial carcinoma (UTUC) are disclosed. The methods involve local delivery of liposomal formulations of chemotherapeutic agents into the ureter and/or renal pelvis via retrograde or antegrade catheter access. The invention is defined in the claims.

### Background of the Invention

Upper tract urothelial carcinoma (UTUC) is a relatively uncommon type of cancer, accounting for only 5-10% of all urothelial cancers. (Leow et al., 2014, Suriano et al., 2014, Roupret et al., 2015). The estimated annual incidence of UTUC in Western countries is about two cases per 100,000 inhabitants (or 6000 cases/year). (Roupret et al., 2015). UTUCs have a peak incidence in people aged 70-90 years, and they are three times more common in men. In 17% of cases of the disease, concurrent bladder cancer is present. Recurrence in the bladder occurs in 22-47% of UTUC patients, compared with 2-6% in the contralateral upper tract. (Hung et al., 2017). Sixty percent of UTUCs are invasive at diagnosis, compared with 15-25% of bladder tumors. (Leow et al., 2014, Roupret et al., 2015).

Treatment of UTUC in high-risk patients may require radical nephroureterectomy (RNU). On the other hand, low-risk cases can involve conservative management, which can be considered when the contralateral kidney is functional. Kidney-sparing surgery (KSS) for low-risk UTUC allows avoiding the morbidity associated with open radical surgery, without compromising oncological outcomes and kidney function. In addition, conservative management can also be considered in all imperative cases (i.e., in patients with renal insufficiency or solitary functional kidney). The most common KSS approaches include ureteroscopic retrograde tumor ablation, percutaneous anterograde tumor ablation, or segmental ureterectomy.

WO 2016/172343 teaches drug-coated balloon catheters for non-vascular strictures, which can also be coated with paclitaxel. WO 2016/134066 teaches liposomal formulations comprising docetaxel. WO 2016/151595 refers to ultrasonic urinary bladder drug delivery, which can be used in combination with a balloon catheter. JP-A 2015/164553 teaches a medical device that has a layer containing a therapeutic agent, which may be paclitaxel. US-A 2009/171241 refers to a medical device comprising a transurethral catheter assembly. US-A 2014/172118 teaches an implantable device comprising a composition comprising triacetin and dimethyl sulfoxide and a bioactive, which can be an anticancer agent. US-A 2009/017105 teaches proliposomal and liposomal compositions of poorly water-soluble drugs. US 9,445,995 teaches a method for preparing a proliposomal powder ispersion containg testosterone, cholesterol and at least one phospholipid. WO 2017/120586 teaches proliposomal powder dispersions comprising taxane, cisplatin or paclitaxel in combination with dipalmitoyl phosphatidylcholine and dimyristoyl phosphatidylglycerol sodium.

Endoscopic ablation of UTUC using ureteroscopy can be considered in highly selected cases and in the following situations: laser generator and alligator type endoscopic forceps are available for biopsies; flexible and rigid ureteroscope; when the patient is informed of the need for closer, more stringent, surveillance; and when a complete tumor resection is strongly advocated. However, endoscopic access to remote anatomical regions of the renal pelvis may limit the ability to surgically ablate or biopsy. In addition, with pure endoscopic management, there is a risk of under staging and under grading the tumor. Furthermore, recurrence rates for of renal pelvic and ureteral tumors treated with ureteroscopy are high, 33-35% and 31-32%, respectively.

To combat tumor recurrence, surgical management of UTUC can be accompanied by postoperative instillation therapy using antitumor chemotherapeutic agents. In UTUC, inter-luminal instillation of MMC has been reported following KSS with promising results (Aboumarzouk et al., 2013). However, because of the difficulties in ureter access and achieving adequate drug dwell time, topical chemotherapy administration after endoscopic management has a minimal effect on disease recurrence (Keeley et al., 1997, Goel et al., 2003, Aboumarzouk et al., 2013, Hutchinson et al., 2016).

The continued recurrence of urothelial tumors even after surgical removal and local postoperative chemotherapy points to a need for additional treatment measures for UTUC patients. Inter-luminal delivery into the upper urinary tract poses a unique challenge given the aqueous environment of the ureteral contents, expulsive peristalsis, and the difficulties in penetrating the urothelial barrier.

Thus there is currently a need for antitumor chemotherapeutic agents for use in endoscopic methods of treating UTUC that involve instillation therapy using antitumor chemotherapeutic agents. There is also a need for chemotherapeutic agent for use in methods of treating UTUC where the dwell time of the chemotherapeutic agent is prolonged, thus allowing increased exposure of the urothelial carcinoma to the chemotherapeutic agents. Also needed is a chemotherapeutic agent for use in methods that use stable liposomal formulations of a chemotherapeutic agent, where the chemotherapeutic agent does not precipitate in the urine pH, and where the formulations allow for the agent to at least partially adhere to the urothelial wall of the ureter and/or renal pelvis affected by UTUC. Such chemotherapeutic agent for use in methods can minimize side effects of the therapy while increasing or maintaining its efficacy.

### Summary of Invention

Herein disclosed are compositions and methods to treat upper tract urothelial carcinoma. A disclosed method for locally delivering a chemotherapeutic agent to an upper tract urothelial carcinoma (UTUC): places a balloon catheter with a working channel, via retrograde or antegrade ureteral access into the ureter and/or renal pelvis; inflates the catheter balloon such that the ureter is temporarily obstructed; instills a liquid liposomal formulation comprising a chemotherapeutic agent into the working channel of the catheter; and allows the instilled liposomal formulation to dwell in the ureter and/or renal pelvis for a time sufficient to allow at least a portion of the liposomal formulation to adhere to the urothelial wall. The invention is defined in the claims.

### Brief Description of the Figures

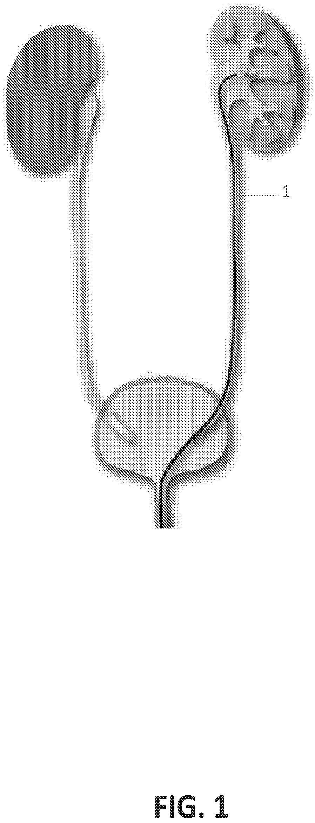

Fig. 1 depicts delivery of a liposomal formulation of a chemotherapeutic formulation into the ureter and renal pelvis via retrograde access.

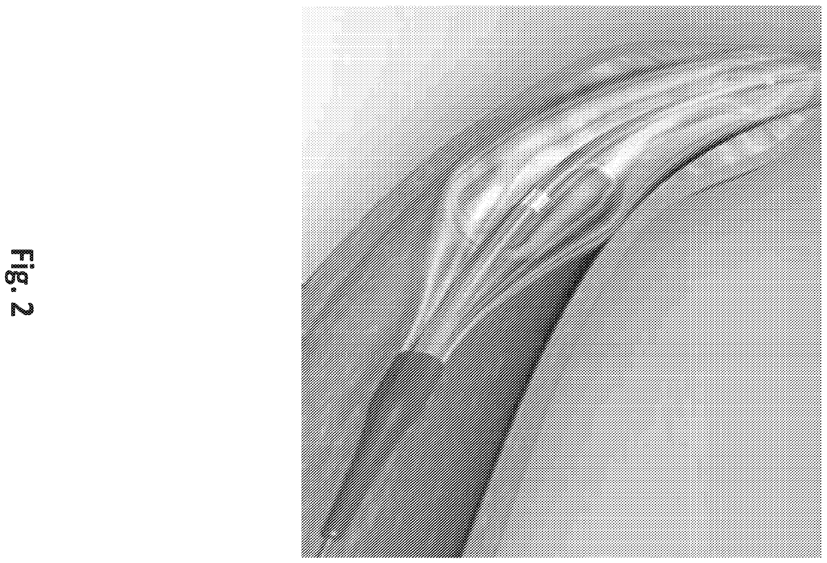

Fig. 2 shows delivering a chemotherapeutic agent using a balloon occlusion of the ureter.

### Detailed Description of the Invention

Disclosed are methods for locally delivering a chemotherapeutic agent to an upper tract urothelial carcinoma (UTUC).The disclosed methods involve placing a balloon catheter that has a working channel and a balloon into the ureter/renal pelvis via retrograde (or antegrade) upper urothelial tract access; inflating the catheter balloon to temporarily obstruct the ureter; infusing (instilling) a liquid liposomal formulation with the chemotherapeutic agent into the working channel of the catheter; and allowing the infused liposomal formulation to dwell in the ureter and/or renal pelvis for a period of time sufficient to allow at least a portion of the formulation to adhere to the urothelial wall. In disclosed methods, the dwell time is from about 1 to about 120 minutes. For example, the liposomal formulation can be allowed to dwell for at least about 10 minutes, about 15 minutes, about 20 minutes, about 30 minutes, about 45 minutes, about one hour or about 2 hours. In the disclosed methods, the infused chemotherapeutic-agent liposomal formulation comes into contact with and adheres to the urothelial wall while it is instilled and dwells in the ureter and/or renal pelvis. The invention is defined in the claims.

The efficacy of local chemotherapy instillations can be improved by using drug formulations optimized for the unique environment of the urinary tract and capable of penetrating the urinary epithelium. To that end, a proliposomal formulation is designed to efficiently penetrate the urinary epithelium and deliver efficacious levels of drug to the ureter. Upon reconstitution with an aqueous medium, the proliposomal formulation turns into a liposomal formulation with increased aqueous solubility and, thus, results in improved exposure directly to the upper urinary tract via catheter access.

Any catheter that is suitable for retrograde or antegrade ureteral access can be used. The catheter can be a rigid or, preferably, a flexible balloon catheter. Flexible ureteroscopy is preferable to rigid when, for example, the tumor is located in the renal pelvis, distal ureter, mid-ureter, or the proximal ureter. In a method as disclosed herein, the catheter is a ureteral occlusion catheter. The catheter can be, for example, a Fogarty balloon catheter, a Boston Scientific^{®} Equalizer Balloon Catheter^{®}, or Boston Scientific^{®} Occlusion Balloon Catheter^{®}. Inflation of a balloon catheter allows to prolong the dwell time of the infused chemotherapeutic-agent formulation. Preferably, a catheter with a distal tip inflatable balloon via endoscopic retrograde access to the ureter is utilized.

The catheter device can, for example, be made of silicone or PVC, have a diameter of about 3.2 French, internal diameter of 0.38, and have a 1-cm elongated oval balloon. The balloon pressure can be adjusted as needed, and can, for instance, reach a maximum of 10 mmHg. The catheter device can be inserted and positioned with assistance of an imaging technique, such as fluoroscopy, enhanced with a radiological contrast.After positioning, the balloon is inflated to a sufficient volume to occlude the ureter and a liposomal formulation is instilled directly into the proximal ureter.

Disclosed methods involve ureteroscopic infusion (instillation) of a liposomal formulation of a chemotherapeutic agent to treat the UTUC tumor. It is generally disclosed that any chemotherapeutic agent known in the art to treat UTUC may be used. The chemotherapeutic agent may be, for example, a taxane (such as paclitaxel (according to the present invention in the content range as claimed), docetaxel (not according to the present invention) or cabazitaxel (not according to the present invention)) or cisplatin (according to the present invention in the content range as claimed).

A chemotherapeutic agent for use in treatment of UTUC, such as paclitaxel (according to the present invention in the content range as claimed) or cisplatin (according to the present invention in the content range as claimed), is first formulated as a proliposomal formulation to be used according to the invention. A liposomal formulation can be prepared from the proliposomal formulation prior to instillation into the ureter and/or renal pelvis by adding an aqueous solution to the proliposomal formulation. The aqueous solution may be any used in the art for instillation, such as, for example, sterile water, saline, of a buffer.

The volume of the liposomal formulation can be adjusted as needed, depending, for example, on the size and position of the UTUC tumor. The volume of the infused formulation can be from about 1 mLto about 50 mL. For example, the disclosed methods can be performed by instilling about 1 mL, about 2 mL, about 3 mL, about 4 mL, about 5 mL, about 6 mL, about 7 mL, about 8 mL, about 9 mL, about 10 mL, about 11 mL, about 12 mL, about 13 mL, about 14 mL, about 15 mL, about 20 mL, about 25 mL, about 30 mL, about 35 mL, about 40 mL, about 45 mL or about 50 mL of a liposomal formulation that comprises a chemotherapeutic agent through a ureteral access balloon occlusive catheter into the ureter and/or renal pelvis.

The concentration of a chemotherapeutic agent in the liposomal formulation can be adjusted as needed, in order to deliver the desired dosage of the chemotherapeutic agent. For example, the dose of administered paclitaxel can range from about 0.5 mg/kg to about 20 mg/kg, and can be, for example, about 0.5 mg/kg, about 1 mg/kg, about 1.5 mg/kg, about 2 mg/kg, about 2.5 mg/kg, about 5 mg/kg, about 10 mg/kg, or about 15 mg/kg.

### Proliposomal Formulations

The formulations for use in methods locally deliver a chemotherapeutic agent to an UTUC tumor that involves instilling a liposomal formulation of a chemotherapeutic agent into the ureter and/or renal pelvis. A liposomal formulation is prepared using a proliposomal formulation of a chemotherapeutic agent.

Suspensions of liposomes for use in methods of treating UTUC administer the suspensions of liposomes, including nanosized poorly water-soluble drug-incorporated liposomal vesicles that incorporate a chemotherapeutic agent, or a combination of active agents. The liposomes may be prepared by hydrating proliposomal powder dispersions formed by a cast-film method, as described in Examples 1-4, herein, and in U.S. Pat. Nos.: 9,445,995 and 6,759,058. Liposome formulations and proliposomal formulations may also be prepared by an organic solvent-free method similar to the one described in Examples below.

Generally, the lipid component of a liposome includes a phospholipid subcomponent, which may consist of phospholipid molecules belonging to a single species, or consist of combinations of various species. A phospholipid component of a proliposomal powder dispersion disclosed herein can be any pharmaceutically acceptable phospholipid and mixtures of such phospholipids. Natural as well as synthetic phospholipids and phospholipid derivatives are accommodated, and referred to generally as phospholipids.

Phospholipids are molecules that have two primary regions, a hydrophilic head region comprised of a phosphate of an organic molecule and one or more hydrophobic fatty acid tails. Naturally-occurring phospholipids generally have a hydrophilic region comprised of choline, glycerol and a phosphate and two hydrophobic regions comprised of fatty acid. When phospholipids are placed in an aqueous environment, the hydrophilic heads come together in a linear configuration with their hydrophobic tails aligned essentially parallel to one another. A second line of molecules then aligns tail-to-tail with the first line as the hydrophobic tails attempt to avoid the aqueous environment. To achieve maximum avoidance of contact with the aqueous environment, i.e., at the edges of the bilayers, while at the same time minimizing the surface area to volume ratio and thereby achieve a minimal energy conformation, the two lines of phospholipids, known as a phospholipid bilayer or a lamella, converge into a liposome. In doing so, the liposomes (or phospholipid spheres) entrap some of the aqueous medium, and whatever may be dissolved or suspended in it, in the core of the sphere. This includes components of a proliposomal powder dispersion disclosed herein, such as testosterone and other components.

Examples of suitable phospholipids that may be used in a proliposomal powder dispersion disclosed herein include distearoyl phosphatidylcholine (DSPC), dipalmitoyl phosphatidylcholine (DPPC), dimyristoyl phosphatidylcholine (DMPC), egg phosphatidylcholine (egg-PC), soy phosphatidylcholine (soy-PC), dimyristoyl phosphatidylglycerol sodium (DMPG), 1,2-dimyristoyl-phosphatidic acid (DMPA), dipalmitoylphosphatidylglycerol (DPPG), dipalmitoyl phosphate (DPP), 1,2-distearoyl-sn-glycero-3-phospho-rac-glycerol (DSPG), 1,2-distearoyl-sn-glycero-3-phosphatidic acid (DSGPA), phosphatidylserine (PS), and sphingomyelin (SM). The proliposomal powder dispersions disclosed herein may also comprise individual combinations of any of the aforementioned phospholipids.

Proliposomal formulations, i.e., proliposomal powder dispersions, are mixtures of at least (a) a drug, which is typically a chemotherapeutic agent, (b) a first phospholipid, and (c) a second phospholipid, dispersed one in another, and which forms a liposome upon contact with an aqueous solution. It is disclosed that a proliposomal powder dispersion may contain (a), (b), and (c) in weight/weight ratios of (a) : (b) : (c) that range from (1.0) : (1.0 - 3.0) : (0.1 - 1.5) (specific ranges according to the present invention are defined in the claims. A proliposomal powder dispersion may also contain (d), cholesterol, in addition to ingredients (a-c). Thus, a proliposomal formulation may contain (a), (b), (c), (d) in weight/weight ratios of (a) : (b) : (c) (d) that range from (1.0) : (1.0 - 3.0) : (0.1 - 1.5) : (0.1 - 2.0) (specific ranges according to the present invention are defined in the claims).

Prior to delivery, the proliposomal formulation is hydrated in water or an aqueous solution, such that liposomes, encapsulating the chemotherapeutic drug within the liposome are formed. In addition to water or an aqueous solution, the resulting liposome suspension may contain a lyo/cryoprotectant, such as sucrose, trehalose, or mannitol. Typically, the lyo/cryoprotectant component of a liposomal formulation is in a w/w ratio with the drug component (lyo/cryoprotectant : drug from about (0.5 : 1.0) to (4.5 : 1.0). For example, a liposome suspension for use in methods of treatment may be prepared by mixing a proliposomal powder, containing (a) a drug, which is typically a chemotherapeutic agent, (b) a first phospholipid, and (c) a second phospholipid and (e) a lyo/cryoprotectant (1.0) : (1.0 - 3.0) : (0.1 - 1.5) : (0.5-4.5) (specific ranges according to the present invention are defined in the claims).

The liposomal formulations used in the invention and proliposomal formulations can accommodate various drugs and active agents. With respect to formulations for use in treating UTUC, the invention accommodates, but is not limited to, taxanes, including paclitaxel (according to the present invention in the claimed context), and docetaxel, cabazitaxel, DJ-927, TPI 287, larotaxel, ortataxel, DHA-paclitaxel, cartazotaxel, and tesetaxel (each not according to the present invention), and cisplatin (according to the present invention in the claimed context).

For example, a proliposomal powder dispersion that contains a taxane derivative drug, ("Propliposmal Intravesical Taxane (PLIT) formulation"), may contain (a) a taxane, (b) a first phospholipid, and (c) a second phospholipid. The liposome suspension of the present invention is defined in the claims. In various embodiments, a proliposomal powder dispersion contains (a), (b), and (c) in weight/weight ratios of (a) : (b) : (c) selected from (1.0) : (1.0 - 3.8) : (0.2 - 1.5); or any ratio therein. For example, in various embodiments of a proliposomal dispersion, the weight/weight ratios of (a) : (b) : (c) may be (1.0) : (3.15) : (1.00); or (1.0) : (3.20) : (1.05); or (1.0) : (3.25) : (1.10); (1.0) : (1.43) : (0.567) ratios of DMPC : DMPG : Paclitaxel, respectively, and 15 mg of mannitol, in 1 ml of sterile water, or any ratio therein.

As indicated above, a proliposomal powder dispersion described herein may also contain cholesterol, in addition to a taxane, a first phospholipid, and a second phospholipid. The liposome suspension of the present invention is defined in the claims. Thus, in various embodiments of a proliposomal powder dispersion used in the invention, it contains weight/weight ratios of (a) : (b) : (c) : (d) selected from (1.0) : (1.0 - 3.8) : (0.4 - 1.5) : (0.5 - 1); or any ratio therein. It follows then, that in certain embodiments of a proliposomal dispersion, in which (a) is paclitaxel, the first phospholipid, (b) is DMPC, the second phospholipid, (c), is DMPG, and (d) is cholesterol, the weight/weight ratios of (a) : (b) : (c) : (d) are (1.0) : (3.40) : (1.25) : (0.70); or (1.0) : (3.45) : (1.30) : (0.75); or (1.0) : (3.50) : (1.35) : (0.80); or any ratio therein. The specific ingredients are defined in the claims.

Alternatively, a proliposomal and liposomal formulation used in the invention may, for example, contain Cis-diamminedichloroplatinum(II), commonly known as cisplatin. It follows then, that a proliposomal powder dispersion used in the invention that contains cisplatin, ("Proliposomal Intravesical Cisplatin (PLIC) formulation"), may contain (a) cisplatin, (b) a first phospholipid, and (c) a second phospholipid. The liposome suspension of the present invention is defined in the claims. In various embodiments, a proliposomal powder dispersion contains (a), (b), and (c) in weight/weight ratios of (a) : (b) : (c) selected from (1.0) : (2.5 - 4.5) : (1.5 - 2.5); or any ratio therein. For example, in various embodiments of a proliposomal dispersion, the weight/weight ratios of (a) : (b) : (c) may be (1.0) : (2.7) : (1.2); or (1.0) : (2.75) : (1.21); or (1.0) : (2.76) : (1.22); or (1.0) : (2.77) : (1.2); or (1.0) : (2.78) : (1.22); or any ratio therein. It follows then, that in certain embodiments of a proliposomal dispersion, in which (a) is cisplatin, the first phospholipid, (b) is DMPC, and the second phospholipid, (c), is DMPG, the weight/weight ratios of (a) : (b) : (c) are (1.0) : (2.7) : (1.2); or (1.0) : (2.75) : (1.21); or (1.0) : (2.76) : (1.22); or (1.0) : (2.77) : (1.2); or (1.0) : (2.78) : (1.22); or any ratio therein. The specific ingredients are defined in the claims.

In other embodiments of a proliposomal dispersion, the weight/weight ratios of (a) : (b) : (c) may be (1.0) : (4.1) : (2.1); or (1.0) : (4.15) : (2.25); or (1.0) : (4.16) : (2.26); or (1.0) : (4.17) : (2.27); or any ratio therein. It follows then, that in certain embodiments of a proliposomal dispersion, in which (a) is cisplatin, the first phospholipid, (b) is DMPC, and the second phospholipid, (c), is DMPG, the weight/weight ratios of (a) : (b) : (c) may be (1.0) : (4.1) : (2.1); or (1.0) : (4.15) : (2.25); or (1.0) : (4.16) : (2.26); or (1.0) : (4.17) : (2.27); or any ratio therein. The specific ingredients are defined in the claims.

As with PLIT formulations, a proliposomal powder dispersion described herein may contain cholesterol in addition to cisplatin, a first phospholipid, and a second. The liposome suspension of the present invention is defined in the claims.. Thus, in various embodiments of a proliposomal powder dispersion according to the invention, it contains weight/weight ratios of (a) : (b) : (c) : (d) selected from (1.0) : (2.5 - 4.5) : (1.5 - 2.5) : (0.5 - 1); or any ratio therein. It follows then, that in certain embodiments of a proliposomal dispersion, in which (a) is cisplatin, the first phospholipid, (b) is DMPC, the second phospholipid, (c), is DMPG, and (d) is cholesterol, the weight/weight ratios of (a) : (b) : (c) : (d) are (1.0) : (2.7) : (1.2) : (1.6); or (1.0) : (2.75) : (1.21) : (1.65); or (1.0) : (2.76) : (1.22) : (1.7); or (1.0) : (2.77) : (1.2) : (1.75); or (1.0) : (2.78) : (1.22) : (1.8); or any ratio therein.

### Pharmaceutical formulations and dosage forms

The invention accommodates various pharmaceutical excipients which can be combined with a proliposomal powder dispersion to create a pharmaceutical formulation or dosage form. Paclitaxel is dissolved along with lipids in ethanol and a thin film was casted using a rotary flash evaporator. The dried film was hydrated using normal saline or water or any pharmaceutically acceptable solvent. This provides us with a liposomal dispersion. This dispersion was extruded using an Emulsiflex^{™}-C5 (Avestin, Canada) or similar high pressure homogenizer or suitable instruments known in literature than can bring in the desired size reduction of in the range of 200 nm. To this liposomal dispersion, one can add suitable excipients externally and subject to lyophilization to obtain a proliposomal powder, i.e., excipients are added "externally." For example, a proliposomal powder dispersion may be admixed with at least one pharmaceutically acceptable excipient. Exemplary pharmaceutically acceptable excipients include, but are not limited to: (a) cryoprotectant, fillers or extenders, such as, for example, starches, lactose (e.g., lactose monohydrate), sucrose, glucose, mannitol, trehalose and silicic acid; (b) binders, such as, for example, cellulose derivatives, including hydroxypropyl methyl cellulose, which is available commercially as Benecel^{™}, hydroxypropyl cellulose, which is available commercially as Klucel^{™} (Ashland Inc - Covington, KY), starch, aliginates, gelatin, polyvinylpyrrolidone, sucrose, and gum acacia, (c) absorption accelerators, such as, for example, quaternary ammonium compounds, In some embodiments, the pharmaceutically acceptable excipients are used in any suitable amount for the particular excipient.

The disclosed methods for delivery of a chemotherapeutic agent into the ureter and/or renal pelvis to treat UTUC can be used as an adjuvant therapy, or additional treatment, to other methods of UTUC treatment. For example, the disclosed methods of instilling a liposomal formulation of a chemotherapeutic agent can be adjuvant to ureteroscopic ablation/resection of UTUC. Thus the methods may include intrarenal or ureteral infusion of liposomal formulated chemotherapy for transitional cell carcinoma.

The liposomal formulation for use in a method according to any one of the proceeding claims, wherein the liposomal formulation is instilled two to five times, repeated twice a week, once a week, every other week, or every four weeks.

### Examples

The following Examples 1-4 describe the preparation of Proliposomal Intravesical Paclitaxel (PLIP) formulations PLIP-003, PLIP-006, PLIP-021, and PLIP-023, respectively. The preparations of the foregoing PLIP formulations were performed by dissolving all of the drug and lipid ingredients together for each formulation, as described in Tables 1-4, respectively, in 10 mL of ethanol in a 500 mL round bottom flask by placing in a water-bath at 50°C. A thin film was casted from the lipid ingredients and ethanol mixture by drying it using a rotary flash evaporator (Buchi) under reduced pressure. The film was completely dried overnight at room temperature under reduced pressure (150~200 mbar). The film was then hydrated using 20 mL of saline by placing the flask on a water-bath at 50°C. The flask was rotated using the Buchi rotary flash evaporator, which resulted in the formation of liposomal dispersion. The dispersion was then homogenized at room temperature under high pressure using a Nano DeBee^{®} high pressure homogenizer to yield unilamellar liposomes in the size range of 100-200 nm size particles. The prepared dispersion was then extruded using an EmusiFlex^{®}-C5 homogenizer. Extrusion was carried out using a polycarbonate membrane with pore diameters that decreased in size from 1 µm to 0.2 µm. To the final extrusion, the amount of mannitol, as described in Tables 1-3, respectively, was added, and the mix was lyophilized to obtain proliposomes.

Example 1. PLIP-003

**Table 1**

| **Ingredients** | **Qty** |
|---|---|
| Paclitaxel (mw = 853.9 Da) | 24 mg |
| DMPG (mw = 688.9 Da; Tc = 23 °C) | 25.2 mg |
| DMPC (mw = 677.9 Da; Tc = 24 °C) | 77.5 mg |
| Mannitol | 100 mg |

Example 2. PLIP-006

**Table 2**

| **Ingredients** | **Qty** |
|---|---|
| Paclitaxel (mw = 853.9 Da) | 25.2 mg |
| DMPG (mw = 688.9 Da; Tc = 23 °C) | 33.8 mg |
| DMPC (mw = 677.9 Da; Tc = 24 °C) | 84.4 mg |
| Cholesterol (mw = 386.65 Da) | 20.1 mg |
| Mannitol | 27 mg |

Example 3. PLIP-021

**Table 3**

| **Ingredients** | **Qty** |
|---|---|
| Paclitaxel | 27.4 mg |
| DMPG | 12.2 mg |
| DMPC | 90.4 mg |
| Mannitol | 50 mg |

Example 4. PLIP-023

**Table 4**

| **Ingredients** | **Qty** |
|---|---|
| Paclitaxel | 25.2 mg |
| DMPG | 18.2 mg |
| DMPC | 90.4 mg |
| Mannitol | 50 mg |

Example 5. An alternative method for the preparation of nanosized poorly water-soluble drug-incorporated liposomal vesicles was performed as follows:
1. Lipid ingredients, such as DMPC, and DMPG were weighed and transferred into an aqueous medium; 2. The aqueous medium was kept at higher temperature than the Tc/Tg of the lipid ingredients; 3. The lipids were hydrated by either allowing the lipid mixture to stand, or by stirring, mixing, and/or homogenization; 4. The poorly water-soluble drug, such as a modified taxane, such as paclitaxel, or a platinum-containing drug, such as cisplatin, was added to the lipid dispersion, and the mixture of drug and lipid was allowed to continue stirring; 5. In order to obtain liposomes, the lipid+drug containing dispersion was homogenized at high pressure and at a temperature higher than the Tc/Tg of the lipids. Homogenization continued until the drug is incorporated into the liposome. Drug incorporation was confirmed by observing the liposomes under a microscope for absence of any drug crystals; 6. Once the drug was incorporated, subsequent homogenization was performed slightly above, at or below the Tc/Tg of the lipid(s) in order to obtain nanosized drug incorporated liposomal vesicles; and 7. A suitable cryo/lyporotectant was added to the liposomal vesicles, followed by lyophilization to obtain drug-loaded proliposomes.

Advantages of the foregoing alternative method of preparing nanosized drug-incorporated liposomal vesicles include: no requirement for use of organic or harsh solvents, such as ethanol, chloroform, and ether; a simple process involving only aqueous medium; a lesser number of unit operations; a lesser number instruments involved in the process; significantly less time is required to obtain drug incorporated liposomal vesicles, as compared to the cast-film method described in Examples 1-4, (two hours preparation time, as compared to two days); and It is a simple, rapid and economical process.

Example 6. PLIP-001. The alternative method for the preparation of nanosized poorly water-soluble drug-incorporated liposomal vesicles, as described in Example 5, was used to prepare paclitaxel formulation PLIP-001, which included the ingredients described in Table 5. The amounts of the PLIP ingredients could be adjusted in proportion the amount of PTX, on a weight/weight ratio basis.

**Table 5.**

| **Ingredients** | **Qty** |
|---|---|
| Paclitaxel | 6 mg |
| DMPG | 3.4 mg |
| DMPC | 8.6 mg |
| Mannitol | 15 mg |
| Sterile water | 1 mL |

Example 7. Ex-vivo assay of PTX delivery to ureter tissue.

A freshly obtained kidney and ureter is rinsed/irrigated with a buffer, such as Phosphate Buffered Saline. The ureter is cannulated with the catheter. The balloon of the catheter is inflated and a purse-string suture is used to secure the catheter. Liposomal formulation containing PTX is directly instilled into the ureter. The volumes of liposomal formulation in the assay are: about 1 mL, about 2 mL, about 3 mL, about 4 mL, about 5 mL, about 6 mL, about 7 mL, about 8 mL, about 9 mL, about 10 mL, about 11 mL, about 12 mL, about 13 mL, about 14 mL, about 15 mL, about 20 mL, about 25 mL, about 30 mL, about 35 mL, about 40 mL, about 45 mL or about 50 mL. The formulation is allowed to remain in the ureter for a specified dwell time: about 10 min, about 20 min, about 30 min, about 1 hr and about 2 hr. The liposomal formulation is drained, and the ureter and kidney are sectioned. PTX is assayed at various tissue depths (0-4000 microns), including measurement of PTX in kidney tissue, for the purpose of assessing transfer kinetics from the liposomal PTX formulation to the ureteral and kidney tissue.

### References

Leow JJ, Martin-Doyle W, Fay AP, Choueiri TK, Chang SL, Bellmunt J. A systematic review and meta-analysis of adjuvant and neoadjuvant chemotherapy for upper tract urothelial carcinoma. Eur Urol. 2014 Sep; 66(3): 529-41.
Roupret M, Babjuk M, Comperat E, Zigeuner R, Sylvester RJ, Burger M, et al. European Association of Urology Guidelines on Upper Urinary Tract Urothelial Cell Carcinoma: 2015 Update. Eur. Urol. 2015 Nov; 68(5): 868-79.
Suriano F, Brancato T. Nephron-sparing Management of Upper Tract Urothelial Carcinoma. Rev Urol. 2014; 16(1): 21-8.
Hung SC, Wang SS, Yang CK, Li JR, Cheng CL, Ou YC, et al. Comparison of Efficacy of Adjuvant MEC (Methotrexate, Epirubicin and Cisplatin) and GC (Gemcitabine and Cisplatin) in Advanced Upper Tract Urothelial Carcinoma. Anticancer Res. 2017 Apr; 37(4): 1875-83.
Aboumarzouk OM, Somani B, Ahmad S, Nabi G, Townell N, Kata SG. Mitomycin C instillation following ureterorenoscopic laser ablation of upper urinary tract carcinoma. Urol Ann. 2013 Jul; 5(3): 184-9.
Keeley FX, Jr., Bagley DH. Adjuvant mitomycin C following endoscopic treatment of upper tract transitional cell carcinoma. J Urol. 1997 Dec; 158(6): 2074-7.
Goel MC, Mahendra V, Roberts JG. Percutaneous management of renal pelvic urothelial tumors: long-term followup. J Urol. 2003 Mar; 169(3): 925-9; discussion 9-30.
Hutchinson R, Haddad A, Sagalowsky A, Margulis V. Upper tract urothelial carcinoma: special considerations. Clin Adv Hematol Oncol. 2016 Feb; 14(2): 101-9.
WO 2016/172343.
WO 2016/134066.
WO 2016/151595.
JP-A 2015/164553.
US-A 2009/171241.
US-A 2014/172118.
US-A 2009/017105.
US 9,445,995.
WO 2017/120586.

## Claims

1. A liposome suspension for use in a method of treating upper tract urothelial carcinoma (UTUC), comprising:
A. placing a balloon catheter with a working channel, via retrograde or antegrade ureteral access into the ureter and/or renal pelvis;
B. inflating the catheter balloon such that the ureter is temporarily obstructed;
C. instilling the liposome suspension into the working channel of the catheter; and
D. allowing the instilled liposome suspension to dwell in the ureter and/or renal pelvis for a period of time that is sufficient to allow at least a portion of the liposomes in the liposome suspension to adhere to the urothelial wall,
wherein the liposomes in the liposome suspension comprise either:
(i)
(a) paclitaxel,
(b) dimyristoyl phosphatidylcholine (DMPC), and
(c) dimyristoyl phosphatidylglycerol sodium (DMPG),
wherein the weight ratios of a : b : care 1 : (1 - 3.8) : (0.2 - 1.5); or
(ii)
(a) cisplatin,
(b) dimyristoyl phosphatidylcholine (DMPC), and
(c) dimyristoyl phosphatidylglycerol sodium (DMPG),
wherein the weight ratios of a : b : c are 1 : (2.5 - 4.5) : (1.5 - 2.5); or
(iii)
(a) cisplatin,
(b) dimyristoyl phosphatidylcholine (DMPC),
(c) dimyristoyl phosphatidylglycerol sodium (DMPG), and
(d) cholesterol,
wherein the weight ratios of a : b : c : d are 1 : (2.5 - 4.5) : (1.5 - 2.5) : (0.5 - 1).

2. The liposome suspension for use according to claim 1, wherein the instilled liposome suspension is allowed to dwell in the ureter and/or renal pelvis for at least about 10 minutes, about 15 minutes, about 20 minutes, about 30 minutes, about 45 minutes, about one hour, or about 2 hours.

3. The liposome suspension for use according to claim 1, wherein the catheter is a ureteral occlusion catheter.

4. The liposome suspension for use according to any one of the proceeding claims, wherein the use of the liposome suspension is an adjuvant therapy for the treatment of UTUC.

5. The liposome suspension for use according to claim 4, wherein the delivery of the liposome suspension is adjuvant to ureteroscopic ablation/resection of a UTUC tumor.

6. The liposome suspension for use according to any one of the proceeding claims, wherein the liposome suspension is instilled two to five times, repeated twice a week, once a week, every other week, or every four weeks.

7. The liposome suspension for use of any one of claims 1 to 6, wherein the liposome suspension further comprises a lyo/cryoprotectant, optionally, mannitol.

8. The liposome suspension for use of any one of claims 1 to 7, wherein the liposomes are according to claim 1 (i) and the concentration of paclitaxel is from about 0.5 mg/kg to about 20 mg/kg.

9. The liposome suspension for use of any one of claims 1 to 8, wherein the liposomes are according to claim 1 (i) and have a weight ratio a : b : c of 0.567 : 1 : 1.43.

## Patentansprüche

1. Liposomen-Suspension zur Verwendung in einem Verfahren des Behandelns eines Urothelkarzinoms des oberen Trakts (UTUC), umfassend:
A. Einsetzen eines Ballonkatheters mit einem Arbeitskanal, über einen retrograden oder antegraden ureteralen Zugang in den Harnleiter und/oder das Nierenbecken;
B. Aufblasen des Katheterballons, so dass der Harnleiter vorübergehend blockiert wird;
C. Einbringen der Liposomen-Suspension in den Arbeitskanal des Katheters; und
D. Erlauben der eingebrachten Liposomen-Suspension im Harnleiter und/oder im Nierenbecken für einen Zeitraum zu verbleiben, der ausreicht, zumindest einem Teil der Liposomen in der Liposomen-Suspension zu erlauben, an der Urothelwand zu haften,
wobei die Liposomen in der Liposomen-Suspension entweder umfassen:
(i)
(a) Paclitaxel,
(b) Dimyristoylphosphatidylcholin (DMPC), und
(c) Dimyristoylphosphatidylglycerin-Natrium (DMPG),
wobei die Gewichtsverhältnisse von a : b : c sind 1 : (1 - 3,8) : (0,2 - 1,5); oder
(ii)
(a) Cisplatin,
(b) Dimyristoylphosphatidylcholin (DMPC), und
(c) Dimyristoylphosphatidylglycerin-Natrium (DMPG),
wobei die Gewichtsverhältnisse von a : b : c sind 1 : (2,5 - 4,5) : (1,5 - 2,5); oder
(iii)
(a) Cisplatin,
(b) Dimyristoylphosphatidylcholin (DMPC),
(c) Dimyristoylphosphatidylglycerin-Natrium (DMPG), und
(d) Cholesterin,
wobei die Gewichtsverhältnisse von a : b : c : d sind 1 : (2,5 - 4,5) : (1,5 - 2,5) : (0,5 - 1).

2. Liposomen-Suspension zur Verwendung gemäß Anspruch 1, wobei der eingebrachten Liposomen-Suspension erlaubt wird, für mindestens etwa 10 Minuten, etwa 15 Minuten, etwa 20 Minuten, etwa 30 Minuten, etwa 45 Minuten, etwa eine Stunde oder etwa 2 Stunden im Harnleiter und/oder Nierenbecken zu verbleiben.

3. Liposomen-Suspension zur Verwendung gemäß Anspruch 1, wobei der Katheter ein Harnleiter-blockierender Katheter ist.

4. Liposomen-Suspension zur Verwendung gemäß einem beliebigen der vorhergehenden Ansprüche, wobei die Verwendung der Liposomen-Suspension eine adjuvante Therapie für die Behandlung von UTUC ist.

5. Liposomen-Suspension zur Verwendung gemäß Anspruch 4, wobei die Verabreichung der Liposomen-Suspension adjuvant zur ureteroskopischen Ablation/Resektion eines UTUC-Tumors ist.

6. Liposomen-Suspension zur Verwendung gemäß einem beliebigen der vorhergehenden Ansprüche, wobei die Liposomen-Suspension zwei bis fünf Mal eingebracht wird, zweimal pro Woche, einmal pro Woche, jede zweite Woche, oder alle vier Wochen wiederholt wird.

7. Liposomen-Suspension zur Verwendung nach einem beliebigen der Ansprüche 1 bis 6, wobei die Liposomen-Suspension ferner ein Lyo-/Kryoschutzmittel, optional Mannitol, umfasst.

8. Liposomen-Suspension zur Verwendung nach einem beliebigen der Ansprüche 1 bis 7, wobei die Liposomen gemäß Anspruch 1 (i) sind und die Konzentration an Paclitaxel von etwa 0,5 mg/kg bis etwa 20 mg/kg ist.

9. Liposomen-Suspension zur Verwendung nach einem beliebigen der Ansprüche 1 bis 8, wobei die Liposomen gemäß Anspruch 1 (i) sind und ein Gewichtsverhältnis a : b : c von 0,567 : 1 : 1,43 aufweisen.

## Revendications

1. Une suspension de liposomes pour être utilisée dans une méthode de traitement du carcinome urothélial des voies supérieures (UTUC), comprenant:
A. la mise en place d'un cathéter à ballonnet comprenant un canal de travail, via un accès urétéral rétrograde ou antégrade dans l'uretère et/ou le bassinet du rein;
B. le gonflage du ballonnet du cathéter de façon que l'uretère soit temporairement obstrué;
C. l'instillation de la suspension de liposomes dans le canal de travail du cathéter; et
D. le maintien de la suspension de liposomes instillée dans l'uretère et/ou le bassinet du rein d'y rester pendant une période suffisante pour permettre à au moins une partie des liposomes de la suspension de liposomes d'adhérer à la paroi urothéliale,
dans laquelle les liposomes de la suspension de liposomes comprennent soit:
(i)
(a) du paclitaxel,
(b) de la dimyristoyl phosphatidylcholine (DMPC), et
(c) du dimyristoyl phosphatidylglycérol sodique (DMPG),
dans laquelle les rapports pondéraux de a/b/c sont 1/(1 -3,8) (0,2 1,5) ; ou
(ii)
a) de la cisplatine,
(b) de la dimyristoyl phosphatidylcholine (DMPC), et
(c) du dimyristoyl phosphatidylglycérol sodique (DMPG),
dans laquelle les rapports pondéraux de a/b/c sont 1/(2,5 - 4,5)/(1,5 - 2,5) ; ou
(iii)
a) de la cisplatine,
(b) de la dimyristoyl phosphatidylcholine (DMPC),
(c) du dimyristoyl phosphatidylglycérol sodique (DMPG), et
(d) du cholestérol, dans lequel les rapports pondéraux de a/b/c/d sont 1/(2,5 - 4,5)/(1,5 - 2,5)/(0,5 - 1).

2. La suspension de liposomes pour une utilisation selon la revendication 1, dans laquelle la suspension de liposomes instillée est maintenue dans l'uretère et/ou le bassinet du rein pendant au moins environ 10 minutes, environ 15 minutes, environ 20 minutes, environ 30 minutes, environ 45 minutes, environ une heure, ou environ 2 heures.

3. La suspension de liposomes pour une utilisation selon la revendication 1, dans laquelle le cathéter est un cathéter d'occlusion urétéral.

4. La suspension de liposomes pour une utilisation selon l'une quelconque des revendications précédentes, dans laquelle l'utilisation de la suspension de liposomes est un traitement adjuvant pour le traitement de l'UTUC.

5. La suspension de liposomes pour une utilisation selon la revendication 4, dans laquelle l'administration de la suspension de liposomes est adjuvante pour l'ablation/résection urétéroscopique d'une tumeur UTUC.

6. La suspension de liposomes pour une utilisation selon l'une quelconque des revendications précédentes, dans laquelle la suspension de liposomes est instillée deux à cinq fois, à raison de deux fois par semaine, une fois par semaine, toutes les deux semaines ou toutes les quatre semaines.

7. La suspension de liposomes pour l'utilisation selon l'une quelconque des revendications 1 à 6, dans laquelle la suspension de liposomes comprend en outre un agent lyo/cryoprotecteur, éventuellement, le mannitol.

8. La suspension de liposomes pour l'utilisation selon l'une quelconque des revendications 1 à 7, dans laquelle les liposomes sont telles que dans la revendication 1(i) et la concentration en paclitaxel est d'environ 0,5 mg/kg à environ20 mg/kg.

9. La suspension de liposomes pour l'utilisation selon l'une quelconque des revendications 1 à 8, dans laquelle les liposomes sont tels que dans la revendication 1 (i) et présentent un rapport pondéral a/b/c de 0,567/1/1,43.
